# EUROPEAN PATENT SPECIFICATION

(11) **EP 1 292 628 B1**
(45) Date of publication and mention of the grant of the patent: **09.06.2010**
(21) Application number: 01938454.4
(22) Date of filing: 15.06.2001
(51) Int. Cl.: C08F 20/58, A61F 13/00, A61L 15/24

(54) **PROCESS FOR THE MANUFACTURE OF HYDROGEL COMPOSITIONS AND HYDROGEL COMPOSITIONS MANUFACTURED THEREBY**
VERFAHREN ZUR HERSTELLUNG VON HYDROGELZUSAMMENSETZUNGEN UND DADURCH HERGESTELLTEN HYDROGELZUSAMMENSETZUNGEN
PROCEDE DE PRODUCTION DE COMPOSITIONS D'HYDROGEL ET COMPOSITIONS D'HYDROGEL PRODUITES SELON LEDIT PROCEDE

(30) Priority: 15.06.2000 GB 0014677; 04.07.2000 GB 0016450
(43) Date of publication of application: 19.03.2003
(73) Proprietor: First Water Limited, Marlborough, Wiltshire SN8 2RB (GB)
(72) Inventor: MUNRO, Hugh Semple, Chipping Camden, Warwickshire GL55 6QT (GB); DONNELLY, Michael Joseph, Styvechale Grange, Coventry CV3 6NR (GB); PAGE, Alison, Earlsdon, Coventry GV5 6DF (GB)
(74) Representative: Brown, David Leslie
(86) International application number: PCT/GB2001/002647
(87) International publication number: WO 2001/096422

(56) References cited:
- EP-A- 0 068 189
- US-A- 3 929 741
- US-A- 5 173 302

## Description

### Field of the Invention

The present invention relates to a process for the manufacture ofhydrogel compositions, and more particularly to a process for the manufacture of high water content hydrogel compositions suitable for use in wound and burn dressings, biomedical electrodes and other applications where bioadhesion is required. The invention also relates.to hydrogel compositions manufactured by the said process.

### Background of the Invention

High water content cross-linked hydrogels based on hydrophilic acrylamido polymers have been previously described by Laskey (US Patent No. 3929741). The ability of these materials to imbibe large quantities of aqueous liquid (e.g. water, biological fluids) and to retain their structural integrity was considered to be advantageous in a number ofbiomedical and consumer applications (Laskey, column 3, lines 36 to 57). Wound and burn coverings are mentioned among many other uses, but with no particular preference expressed for them. A number of methods for initiating the polymerisation and cross-linking were mentioned, including the use of irradiation (Laskey, column 5, lines 1 to 12). Details of how to achieve irradiation-initiated polymerisation and cross-linking were not described.

US-A-5 173 302 discloses a polymerisable composition comprising (a) 20-50% of a monofunctional monomer component, at least 75% of the component comprising 2-acrylamido-2-methylpropane sulphonic acid (I) or salt, the balance being selected from acrylic acid, water soluble acrylic functional monomers and vinyl pyrrolidone, (b) 30-50% of a glycol component selected from HO-(C2H40)n-H and HO-(C3H60)m-H (n = 4-16; m = 1-4), (c) 0.02-0.2% of a crosslinking monomer, (d) a free radical polymerization initiator for initiating polymerization of the monofunctional monomer and crosslinking monomer components and (e) a topically or transdermally deliverable drug, at least 60% of the drug being dissolved in the formulation. The composition is curable by UV irradiation. The polymerised matrix gel material is tacky, light to moderately adhesive and leaves little or no residue on the skin when removed. It can be used as an adhesive reservoir for topical or transdermally administered drugs.

EP-A-0 068 189 discloses a crosslinked copolymer (I) which swells in water consisting of (A) 0.5-75 (5-30) wt.% of units from 2-acrylamido-2-methyl propane sulphonic acid or alkali and/or ammonium salts, (B) 23-99.49 (70-95) wt.% of units from (meth)acrylic acid or alkali and/or ammonium salts, and/or acrylamide, and/or vinylpyrrolidone, and (C) 0.01-2 wt.% of units from an at least bifunctional crosslinking agent. The polymerization is UV initiated.

The manufacturing processes described by Laskey suffers from a number of inherent disadvantages. Primarily, the batchwise polymerisation with cross-linking is not suitable for mass-production, and the relatively high levels of cross-linking agent required (greater than about 0.5% by weight) result in a somewhat brittle hydrogel which is difficult to handle.

It is therefore an object of the present invention to provide an improved or at least alternative method for the commercial manufacture of high water content hydrogels, which yields a product having acceptable skin feel and adhesion properties for biomedical use.

### Brief Description of the Invention

The present invention is based on our surprising finding that reduced levels of cross-linking agent (less than about 0.5% by weight) can give rise to a polymerised and cross-linked hydrogel which has an acceptable non-gxeasy "feel", sufficient structural integrity and low brittleness, as well as good processing properties, if the levels of photoinitiator are also reduced to substantially lower levels than used hitherto.

According to a first aspect of the present invention, there is provided a process for the manufacture of a high water content cross-linked sheet hydrogel composition, comprising:
(i) preparing a mixture comprising:
   (1) one or more unsaturated free radically photo-polymerisable monomer capable of polymerisation to ah ydrophilic polymer;
   (2) one or more free radical photoinitiator;
   (3) one or more cross-linking agent comprising a multifunctional unsaturated free radically photo-polymerisable compound; and
   (4) water; and
(ii) irradiating the mixture with light of sufficient intensity and at an appropriate wavelength to polymerise and cross-link the mixture to form the sheet composition;
   wherein all of components (1) to (4) present in the mixture in step (i) are also present in the composition resulting from step (ii), the photoinitatar (2) is present in the mixture in step (i) in an amount between about 0,002% and about 0.05% by weight of the total mixture, the cross-linking agent (3) is present in the mixture in step (i) in an amount less than about 0.5% by weight of the total mixture, and the resultant hydrogel composition comprises more than about 40% by weight of water.

Most preferably, the mixture prepared in step (i) consists essentially of components (1) to (4) and optionally one or more electrolyte and/or one or more organic plasticiser and/or one or more surfactant, with less than about 10% of other additives.

It has been found that the process of the present invention is highly convenient for an industrial scale continuous sheet production method for preparing hydrogel sheets having a thickness in the range of about 0.2 mm to about 2 mm, Such sheets are typically prepared in contact with a release sheer, for example a sheet of plastic or coated plastic (e.g, siliconised plastic) or paper or coated paper (e.g. siliconised paper) at a surface weight of hydrogel in the range of about 0.5 kg/m² to about 2.5 kg/m². Moreover, the process enables high water content cross-linked hydrogel compositions having predetermined combinations of certain important characteristics to be prepared reproducibly on an industrial scale, when the amounts of the photoinitiator and the cross-linker are controlled in the reaction mixture. According to a further aspect, the present invention may be stated to provide the use of a controlled amount of one or more free radical photoinitiator in the range between about 0.002% and about 0.05% by weight of the total mixture and a controlled amount of one or more multifunctional unsaturated free radically photo-polymerisable cross-linking agent in the range less than about 0.5% by weight of the total mixture, in a pre-photopolymerisation mixture comprising also water and one or more unsaturated free radically photo-polymerisable monomer in a process for the manufacture of a high water content cross-Linked hydrogel composition having a desired pre-determined combination of two or more of: non-greasy feel to human skin, structural integrity, low brittleness and good industrial processability in sheet form.

According to a further aspect of the present invention, there is provided a high water content cross-linked hydrogel composition manufactured by the process or use of the present invention. The composition may suitably be present in the form of a sheet having first and second major faces, each of said first and second major faces being in contact with a protective release layer, for example siliconised plastic or paper.

Alternatively, the composition may be present in the form of a sheet having first and second major faces, one of said first and second major faces being in contact with a protective release layer, for example siliconised plastic or paper, and the other of said first and second major faces being in contact with a backing member, suitably a backing member forming part of a wound or burn dressing, a biomedical electrode or another article where a bioadhesive hydrogel layer is to be provided in use between the article and the skin of a patient. Still further, the composition may be present in the form of a sheet having a woven or non-woven fabric, or a net, embedded therein.

The resultant hydrogels are low or moderately cross-linked materials which, surprisingly, have a very acceptable toughness, low brittleness and high structural integrity with generally very good machine cutting/converting performance in subsequent post-processing. More particularly, we have found that the process enables rolls of sheet hydrogel to be formed in excess of 100m Length. In such a process, the irradiation step (ii) will preferably take place with relative movement between an irradiation source and the mixture resulting from step (i), i.e. preferably the complete mixture will not be irradiated simultaneously.

The expression "high water content" used herein refers to hydrogel compositions comprising more than about 40% by weight of water, more particularly above about 50% by weight, and most preferably between about 60% and about 95% by weight.

The expression "monomer" used herein includes ionic and non-ionic monomers and monomer mixtures. Correspondingly, the expression "polymerise", "polymers" and like expressions include both homopolymerisation and copolymerisation, and the products thereof.

The expression "substantially all of components (1) to (4) present in the mixture in step (i) are also present in the composition resulting from step (ii)" used herein is to be understood as meaning that the desired extent of conversion of monomer and cross-linking agent into polymer takes place, but that material is not substantially removed in or by step (ii). It is preferred also that material is not substantially added to or removed from the composition after step (ii), although some degree of conditioning and/or modification may be desirable.

### Detailed Description of the Invention

### Ionic Monomer

Preferably the one or more ionic monomers are 2-acrylamido-2-methylpropane sulphonic acid or an analogue thereof or one of its salts, e.g. an ammonium or alkali metal salt such as a sodium, potassium or lithium salts; acrylic acid or an analogue thereof or one of its salts, e.g. an alkali metal salt such as a sodium, potassium or lithium salt; and/or a polymerisable sulphonate or a salt thereof, e.g. an alkali metal salt such as a sodium, potassium or lithium salt, of acrylic acid (3-sulphopropyl) ester or an analogue thereof. The term "analogue" in this context refers particularly to substituted derivatives of 2-acrylamido-2-methylpropane sulphonic acid, of acrylic acid or of acrylic acid (3-sulphopropyl) ester.

A particularly preferred ionic monomer is a sodium salt of 2-acrylamido-2-methylpropane sulphonic acid, commonly known as NaAMPS, which is available commercially at present from Lubrizol as either a 50% aqueous solution (reference code LZ2405) or a 58% aqueous solution (reference code LZ2405A) and/or acrylic acid (3-sulphopropyl) ester potassium salt, commonly known as SPA. SPA is available commercially in the form of a pure solid from Raschig. The reaction mixture preferably comprises from about 5% to about 40%, and ideally from about 10% to about 40%, by weight of the reaction mixture, of the ionic monomer, with the proviso that the total amount of monomer (1) in the reaction mixture should preferably be in the range of about 5% to about 40% by weight of the total reaction mixture.

### Non-ionic Monomer

In one embodiment of the invention the aforesaid non-ionic water soluble monomer will comprise at least one of acrylamide or a mono- or di-N-alkylacrylamide or an analogue thereof. The term "analogue" in this context refers to non-ionic water soluble monomers containing an alkyl or substituted alkyl group linked to a carbon-carbon double bond via an amido or alkylamido (-CO.NH- or -CO.NR-) function. Examples of such analogues include diacetone acrylamide (N-1,1-dimethyl-3-oxobutylacrylamide), vinyl lactams; N-alkylated acrylamides, N,N-dialkylated acrylamides, N-vinyl pyrrolidone and acryloyl morpholine. N,N-dimethylacrylamide (NNDMA) and/or an analogue thereof is preferred. When present, the non-ionic water soluble monomer may comprise up to about 30%, and ideally up to about 25%, by weight of the reaction mixture, with the proviso that the total amount of monomer (1) in the reaction mixture should preferably be in the range of about 5% to about 40% by weight of the total reaction mixture.

### Cross-linking Agents and Photoinitiators

Conventional cross-linking agents are suitably used to provide the necessary mechanical stability and to control the adhesive properties of the hydrogel. The amount of cross-linking agent required will be readily apparent to those skilled in the art such as from about 0.01% to about 0.5%, particularly from about 0.05% to about 0.4%, most particularly from about 0.08% to about 0.3%, by weight of the total polymerisation reaction mixture. Typical cross-linkers include tripropylene glycol diacrylate, ethylene glycol dimethacrylate, triacrylate, polyethylene glycol diacrylate (polyethylene glycol (PEG) molecular weight between about 100 and about 4000, for example PEG400 or PEG600), and methylene bis acrylamide.

The hydrogel compositions with which this invention is concerned generally comprise, in addition to a cross-linked polymeric network, an aqueous plasticising medium and, optionally, at least one electrolyte, whilst the materials and processing methods used are normally chosen to provide a suitable balance of adhesive and electrical properties for the desired application. One preferred feature of the process of the invention is that, to achieve the desired properties, the final amount of water required in the hydrogel is present in the formulation prior to gellation, i.e. preferably no water is removed from the hydrogel after manufacture and less than about 10% during manufacture.

As described in more detail below (see the section headed "Polymerisation Conditions"), the process of the invention involves free radical polymerisation and the use of a photoinitiator or a combination of photo- and other initiation. Preferably the reaction mixture comprises an amount of photoinitiator of from about 0.003% to about 0.05%, and particularly from about 0.003% to about 0.04%, most preferably from about 0.009% to about 0.02%, by weight of the total polymerisation reaction mixture. Preferred photoinitiators include any of the following either alone or in combination:
Type I-α-hydroxy-ketones and benzilidimethyl-ketals e.g. Irgacure 651. These are believed on irradiation to form benzoyl radicals that initiate polymerisation. Photoinitiators of this type that are preferred are those that do not carry substituents in the *para* position of the aromatic ring. Examples include Irgacure 184 and Daracur 1173 as marketed by Ciba Chemicals, as well as combinations thereof.
Photoinitiators of the following general formula are preferred: 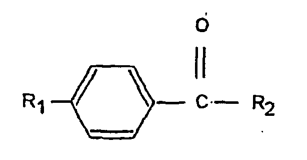 where R₁ can be any of the following:- hydrogen, H₃C-S-, 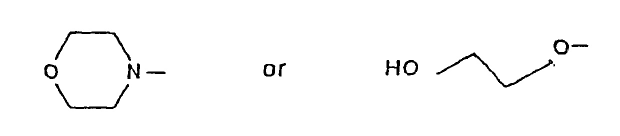 R₁ is most preferably hydrogen.
   R₂ can suitably be any of the following:- 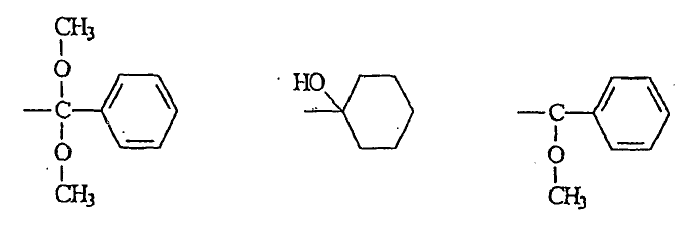 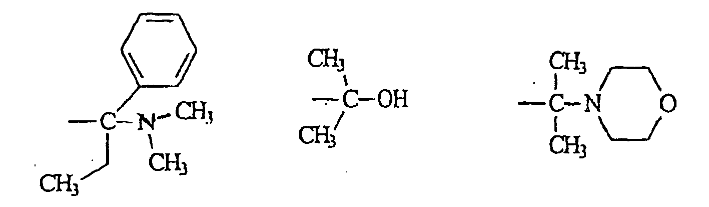 R₂ is most preferably as follows:-

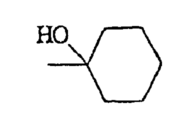

A particularly preferred photoinitiator is 1-hydroxycyclohexyl phenyl ketone; for example, as marketed under the trade name Irgacure 184 by Ciba Speciality Chemicals. Also preferred are Daracur 1173 (2-hydroxy-2-propyl phenyl ketone) and mixtures of Irgacure 184 and Daracur 1173.

### Applications

The compositions described herein may suitably be used in a range of skin contact or covering applications where the composition is brought into contact either with skin or with an intermediary article which interfaces between the composition and the skin. The composition may be unsupported or supported on a backing structure. The compositions may suitably be in the form of sheets, coatings, membranes, composites or laminates. Such applications include patches, tapes, bandages, devices and dressings of general utility or for specific uses, including without limitation biomedical, skin care, personal and body care, palliative and veterinary uses such as, for example, skin electrodes; wound and burn healing; wound and burn management; skin cooling; skin moisturising; skin warming; aroma release or delivery; decongestant release or delivery; pharmaceutical and drug release or delivery; perfume release or delivery; fragrance release or delivery; scent release or delivery; adhesive use, e.g. in skin contacting devices, ostomy and related incontinence devices, and the like. In some fields of application, such as, for example, pharmaceutical delivery devices for the delivery of pharmaceuticals or other active agents to or through mammalian skin, the compositions may optionally contain topical, transdermal or iontophoretic agents and excipients. The compositions may contain penetration-enhancing agents to assist the delivery of water or active agents into the skin. Non-limiting examples of penetration-enhancing agents for use in such applications include methyl oleic acid, isopropyl myristate, Azone ® Transcutol ® and N-methyl pyrrolidone.

The compositions prepared according to the present invention are used in these applications in generally conventional manner, as will be readily understood by those skilled in this art.

### Biomedical Skin Electrodes

A particularly preferred application is in the field of biomedical skin electrodes.

When the hydrogels are intended for use in conjunction with Ag/AgCl medical electrodes, chloride ions are required to be present in order for the electrode to function. Potassium chloride and sodium chloride are commonly used. However any compound capable of donating chloride ions to the system may be used, for example, lithium chloride, calcium chloride, ammonium chloride. The amount that should be added is dependent on the electrical properties required and is typically about 0.5-8% by weight.

In general, an electrolyte (e.g. a salt) will need to be included in the polymerisation reaction mixture in appropriate amounts, when the process is used to manufacture a hydrogel composition for use in an electrode.

The compositions prepared according to the present invention are used in biomedical electrodes in generally conventional manner, as will be readily understood by those skilled in this art.

### Plasticiser

In one embodiment of the invention the one or more organic plasticiser, when present, may suitably comprise any of the following either alone or in combination: at least one polyhydric alcohol (such as glycerol), at least one ester derived therefrom, at least one polymeric alcohol (such as polyethylene oxide) and/or at least one mono- or poly-alkylated derivative of a polymeric alcohol (such as alkylated polyethylene glycol). Glycerol is the preferred plasticiser. An alternative preferred plasticiser is the ester derived from boric acid and glycerol. When present, the organic plasticiser may comprise up to about 45% by weight of the polymerisation reaction mixture.

### Surfactant

Any compatible surfactant may optionally be used as an additional ingredient of the polymerisation mixture. Surfactants can lower the surface tension of the mixture before polymerisation and thus aid processing. Non-ionic, anionic and cationic surfactants are preferred. The surfactant ideally comprises any of the surfactants listed below either alone or in combination with each other and/or with other surfactants. The total amount of surfactant, if present, is suitably up to about 10% by weight of the total polymerisation reaction mixture, preferably from about 0.05% to about 2% by weight, more preferably from about 0.05% to about 1% by weight.

### 1. Non-ionic Surfactants

Suitable non-ionic surfactants include, but are not limited to, those selected from the group consisting of the condensation products of a higher aliphatic alcohol, such as a fatty alcohol, containing about 8 to about 20 carbon atoms, in a straight or branched chain configuration, condensed with about 3 to about 100 moles, preferably about 5 to about 40 moles and most preferably about 5 to about 20 moles of ethylene oxide.

Examples ofsuch non-ionic ethoxylated fatty alcohol surfactants are the Tergitol^{™} 15-S series from Union. Carbide and Brij^{™} surfactants from ICI. Tergitol^{™} 15-S surfactants include C₁₁-C₁₅ secondary alcohol polyethyleneglycol ethers. Brij^{™} 58 surfactant is polyoxyethylene(20) cetyl ether, andBrij^{™} 76 surfactant is polyoxyethylene(10) stearyl ether.

Other suitable non-ionic surfactants include, but are not limited to, those selected from the group consisting of the polyethylene oxide condensates of one mole of alkyl phenol containing from about 6 to 12 carbon atoms in a straight or branched chain configuration, with about 3 to about 100 moles of ethylene oxide. Examples of non-ionic surfactants are the Igepal^{™} CO and CA series from Rhone-Poulenc. Igepal^{™} CO surfactants include nonylphenoxy poly(ethyleneoxy) ethanols. Igepal^{™} CA surfactants include octylphenoxy poly(ethyloneoxy) ethanols.

Another group of usable non-ionic surfactants include, but are not limited to, those selected from the group consisting ofblock copolymers of ethylene oxide and propylene oxide or butylene oxide. Examples of such non-ionic block copolymer surfactants are the Pluronic^{™} and Tetronic^{™} series of surfactants from BASF. Pluronic^{™} surfactants include ethylene oxide-propylene oxide block copolymers. Tetronic^{™} surfactants include ethylene oxide-propylene oxide block copolymers. The balance ofhydrophobic and hydrophilic components within the surfactant together with the molecular weight are found to be important. Suitable examples are Pluronic L68 and Tetronic 1907. Particularly suitable examples are Pluronic L64 and Tetronic 1107.

Still other satisfactory non-ionic surfactants include, but are not limited to, those selected from the group consisting of sorbitan fatty acid esters, polyoxyethylene sorbitan fatty acid esters and polyoxyethylene stearates. Examples of such fatty acid ester non-ionic surfactants are the Span^{™}, Tween^{™}, and Myrj^{™} surfactants from ICI. Span^{™} surfactants include C₁₂-C₁₈ sorbitan monoesters. Tween^{™} surfactants include poly(ethylene oxide) C₁₂-C₁₈ sorbitan monoesters. Myrj^{™} surfactants include poly(ethylene oxide) stearates.

### 2. Anionic Surfactants

Anionic surfactants normally include a hydrophobic moiety selected from the group consisting of (about C₆ to about C₂₀) alkyl, alkylaryl, and alkenyl groups and an anionic group selected from the group consisting of sulfate, sulfonate, phosphate, polyoxyethylene sulfate, polyoxyethylene sulfonate, polyoxyethylene phosphate and the alkali metal salts, ammonium salts, and tertiary amino salts of such anionic groups.

Anionic surfactants which can be used in the present invention include, but are not limited to those selected from the group consisting of (about C₆ to about C₂₀) alkyl or alkylaryl sulfates or sulfonates such as sodium lauryl sulfate (commercially available as Polystep^{™} B-3 from Srepan Co.) and sodium dodecyl benzene sulfonate, (commercially available as Siponate^{™} DS-10 from Rhone-Poulenc); polyoxyethylene (about C₆ to about C₂₀) alkyl or alkylphenol ether sulfates with the ethylene oxide repeating unit in the surfactant below about 30 units, preferably below about 20 units, most preferably below about 15 units, such as Polystep^{™} B-1 commercially available from Stepan Co. and Alipal^{™} EP110 and 115 from Rhone-Poulenc; (about C₆ to about C₂₀) alkyl or alkylphenoxy poly (ethyleneoxy)ethyl mono-esters and di-esters of phosphoric acid and its salts, with the ethylene oxide repeating unit in the surfactant below about 30 units, preferably below about 20 units, most preferably below about 15 units, such as Gafac^{™} RE-510 and Gafac^{™} RE-610 from GAF.

### 3. Cationic Surfactants

Cationic surfactants useful in the present invention include, but are not limited to, those selected from the group consisting of quaternary ammonium salts in which at least one higher molecular weight group and two or three lower molecular weight groups are linked to a common nitrogen atom to produce a cation, and wherein the electrically-balancing anion is selected from the group consisting of a halide (bromide, chloride, etc.), acetate, nitrite, and lower alkosulfate (methosulfate etc.). The higher molecular weight substituent(s) on the nitrogen is/are often (a) higher alkyl group(s), containing about 10 to about 20 carbon atoms, and the lowermolecular weight substituents may be lower alkyl of about 1 to about 4 carbon atoms, such as methyl or ethyl, which may be substituted, as with hydroxy, in some instances. One or more of the substituents may include an aryl moiety or may be replaced by an aryl, such as benzyl or phenyl.

In a preferred embodiment of the invention the surfactant comprises at least one propylene oxide/ethylene oxide block copolymer, for example such as that supplied by BASF Plc under the trade name Pluronic P65 or L64.

### Polymerisation Conditions

In preparing hydrogel compositions in accordance with the invention, the ingredients will be mixed to provide a reaction mixture in the form of an initial pre-gel aqueous based liquid formulation, and this is then converted into a hydrogel by a free radical polymerisation reaction. Photo-polymerisation may be achieved using photoinitiators, optionally together with other initiators, such as heat and/or ionizing radiation. Photoinitiation will usually be applied by subjecting the pre-gel reaction mixture containing an appropriate photoinitiation agent to ultraviolet (UV) light after it has been spread or coated as a layer on siliconised release paper or other solid substrate. The incident UV intensity, at a wavelength in the range from 240 to 420nm, is typically greater than about 10mW/cm². The processing will generally be carried out in a controlled manner involving a precise predetermined sequence of mixing and thermal treatment or history.

The UV irradiation time scale should ideally be less than 60 seconds, and preferably less than 10 seconds to form a gel with better than 95% conversion of the monomers. Those skilled in the art will appreciate that the extent of irradiation will be dependent on a number of factors, including the UV intensity, the type of UV source used, the photoinitiator quantum yield, the amount of monomer present, the nature of the monomer(s) present, the presence of dissolved oxygen, the presence of polymerisation inhibitor, the thickness of the reaction mixture when coated onto the substrate and the nature of substrate onto which the reaction mixture is coated.

### Other additives

Additional polymer(s), typically rheology modifying polymer(s), may be incorporated into the polymerisation reaction mixture at levels typically up to about 10% by weight of total polymerisation reaction mixture, e.g. from about 0.2% to about 10% by weight. Such polymer(s) may include polyacrylamide, poly-NaAMPS, polyethylene glycol (PEG), polyvinylpyrrolidone (PVP) or carboxymethyl cellulose.

Additional functional ingredients may also incorporated in the reaction mixture used in the invention, including antimicrobial agents (e.g. citric acid, stannous chloride) and, for drug delivery applications, pharmaceutically active agents, the latter being designed to be delivered either passively (e.g. transdermally) or actively (e.g. iontophoretically) through the skin. For this purpose, penetration-enhancing agents may also be present in the reaction mixture and resultant hydrogel, as described above in the section headed "Applications".

### Examples of the Invention

The invention will be further described with reference to the following Examples, which should not be understood to limit the scope of the invention.

### EXAMPLE 1

400g of a 50% aqueous solution of sodium-2-acrylamido-2-methylpropane sulphonate (NaAMPS, Lubrizol) was added to 450g water. 150g glycerol was added and the mixture stirred for 30 minutes. A solution of crosslinker and photoinitiator was made by adding 2.3g of IRR280 (PEG400 diacrylate, UCB Chemicals) to 0.012g of photoinitiator, Daracur 1173 (Ciba Specialty Chemicals). This was added to the mixture, which was stirred for 1 hour, covered to exclude light. 50g of the mixture at a coat weight of 1.5kg/sq.m was cured in the laboratory on a tray lined with siliconised paper by passing at a speed of 7m/minute three times under ultra-violet (UV) radiation of 80W/cm from a medium pressure mercury vapour lamp. The cured gel was covered with a siliconised high density polyethylene (HDPE) top liner.

### EXAMPLES 2 to 8

Gels having various photoinitiator levels were made by the procedure described in Example 1 above, substituting the 0.012g of photoinitiator by the amounts shown in Table 1.

**TABLE 1**

| **Example No.** | **1** | **2** | **3** | **4** | **5** | **6** | **7** | **8** |
|---|---|---|---|---|---|---|---|---|
| Daracur 1173 (g) | 0.012 | 0.035 | 0.115 | 0.23 | 0.345 | 0.46 | 0.575 | 0.69 |

Examples 2, 3, 4, 5 and 6 gave acceptable materials; 1, 7 and 8 did not.

### EXAMPLES 9 to 16

Formulations were prepared containing the following parts by weight, as shown in Table 2.

**TABLE 2**

| **Example No.** | **9** | **10** | **11** | **12** | **13** | **14** | **15** | **16** |
|---|---|---|---|---|---|---|---|---|
| 50% NaAMPS solution | 40 | 57 | 45 | 36 | 50 | 40 | 57 | 57 |
| Water | 60 | 43 | 38 | 64 | 50 | 60 | 43 | 43 |
| Pluronic P65 (surfactant) | 0.18 | 0.18 | 0.18 | 0.18 | 0.18 | 0.18 | 0.18 | 0.18 |
| Polyacrylamide (12% solution) | - | - | 17 | - | - | - | - | - |
| Chlorohexidine Gluconate | - | - | - | - | - | - | - | 0.5 |
| Potassium Chloride | - | - | - | - | - | 5 | 4 | - |
| IRR280 | 0.29 | 0.19 | 0.238 | 0.29 | 0.143 | 0.29 | 0.29 | 0.29 |
| Daracur. 1173 | 0.01 | 0.01 | 0.01 | 0.01 | 0.007 | 0.01 | 0.01 | 0.01 |

Polyacrylamide solution available as Glascol W17^{™} Ciba Specialty Chemicals
Chlorohexidine Gluconate and Potassium Chloride from Aldrich
Pluronic P65 from BASF

Examples 9-16 gave acceptable hydrogels.

## Claims

1. A process for the manufacture of a high water content cross-linked sheet hydrogel composition, comprising:
(i) preparing a mixture comprising:
(1) one or more unsaturated free radically photo-polymerisable monomer capable of polymerisation to a hydrophilic polymer;
(2) one or more free radical photoinitiator;
(3) one or more cross-linking agent comprising a multifunctional unsaturated free radically photo-polymerisable compound;
and
(4) water; and
(ii) irradiating the mixture with light of sufficient intensity and at an appropriate wavelength to polymerise and cross-link the mixture to form the sheet composition;
wherein all of the compounds (1) to (4) present in the mixture in step (i) are also present in the composition resulting from step (ii), the photoinitiator (2) is present in the mixture in step (i) in an amount between about 0.002% and about 0.05% by weight of the total mixture, the cross-linking agent (3) is present in the mixture in step (i) in an amount less than about 0.5% by weight of the total mixture, and the resultant hydrogel composition comprises more than about 40% by weight of water.

2. A process according to claim 1, wherein the resultant hydrogel composition comprises more than about 50% by weight of water,

3. A process according to claim 2, wherein the resultant hydrogel composition comprises between about 60% and about 95% by weight of water.

4. A process according to any one of the preceding claims, wherein the mixture prepared in step (i) further comprises one or more electrolyte.

5. A process according to any one of the preceding claims, wherein the mixture prepared in step (i) further comprises one or more organic plasticiser.

6. A process according to claim 5, wherein the organic plasticiser comprises any of the following either alone or in combination: at least one polyhydric alcohol, at least one ester derived therefrom, at least one polymeric alcohol and/or at least one mono- or poly-alkylated derivative of a polymeric alcohol.

7. A process according to claim 5 or 6, wherein the organic plasticiser comprises glycerol or an ester derived from boric acid and glycerol,

8. A process according to any one of the preceding claims, wherein the mixture prepared in step (i) further comprises one or more surfactant.

9. A process according to claim 1, wherein the mixture prepared in step (i) consists essentially of components (1) to (4) and optionally one or more electrolyte and/or one or more organic plasticiser and/or one or more surfactant.

10. A process according to any one or the preceding claims, when carried out in a continuous manner for the production of a continuous hydrogel sheet.

11. A process according to claim 10, wherein the hydrogel sheet has a thickness in the range of about 0.2 mm to about 2 mm.

12. A process according to any one of the preceding claims, wherein the one or more monomer comprises 2-acrylamido-2-methylpropane sulphonic acid, acrylic acid, acrylic acid (3-sulphoproyl) ester, a substituted derivative thereof, or a salt thereof.

13. A process according to any one of the preceding claims, wherein the one or more monomer comprises at least one of acrylamide or a mono- or di-N-alkylacrylamide or an analogue thereof containing an alkyl or substituted alkyl group linked to a carbon-carbon double bond via an amido or alkylamido function.

14. A process according to claim 13, wherein the analogue is diacetone acrylamide, a vinyl lactam, an N-alkylated acrylamide, an N,N-dialkylated acrylamide, N-vinyl pyrrolidone or acryloyl morpholine.

15. A process according to claim 14, wherein the monomer is acryloyl morpholine.

16. A process according to any one of the preceding claims, wherein the polymerisation is a homopolymerisation.

17. A process according to any one of claims 1 to 15, wherein the polymerisation is a copolymerisation.

18. A process according to any one of the preceding claims, wherein the monomer component (1) comprises an ionic monomer in an amount of from 5% to 40% by weight of the polymerisation reaction mixture.

19. A process according to any one of the preceding claims, wherein the monomer component (1) comprises a non-ionic monomer present in an amount of up to 30% by weight of the polymerisation reaction mixture,

20. A process according to any one of the preceding claims, wherein the monomer component (1) is present in the polymerisation reaction mixture in the range of 5% to 40% by weight of the total reaction mixture,

21. A process according to any one of the preceding claims, wherein material is not substantially added to or removed from the composition after step (ii).

22. A process according to any one of the preceding claims, wherein no water is removed from the hydrogel after said manufacture and less than 10% of water is removed during said manufacture.

23. A process according to any one of the preceding claims, wherein the photoinitiator (2) is present in the mixture in step (i) in an amount of from about 0.003% to about 0.05% by weight of the total polymerisation reaction mixture.

24. A process according to claim 23, wherein the photoinitiator (2) is present in the mixture in step (i) in an amount of from about 0.003% to about 0.04% by weight of the total polymerisation reaction mixture.

25. A process according to claim 24, wherein the photoinitiator (2) is present in the mixture in step (i) in an amount of from 0.003% to 0.02% by weight of the total reaction mixture.

26. A process according to claim 23, wherein the photoinitiator (2) is present in the mixture in step (i) in an amount of from about 0.009% to about 0.02% by weight of the total polymerisation reaction mixture.

27. A process according to any one of the preceding claims, wherein the photoinitiator comprises a compound of the following general formula: 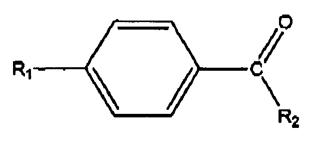 where R₁ is selected from hydrogen, H₃C-S-, 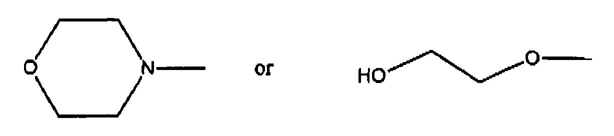 R₂ is selected from

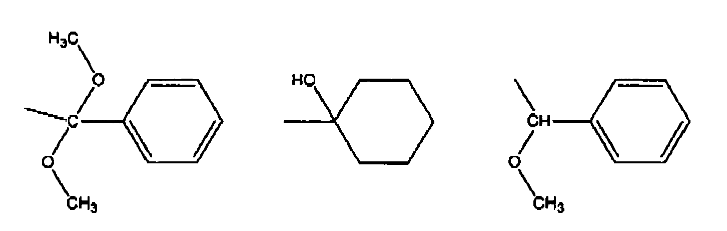

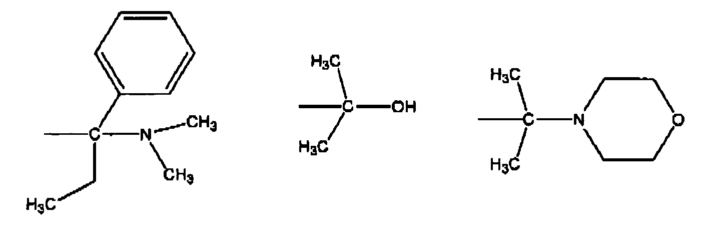

28. A process according to claim 27, wherein R₁ is hydrogen.

29. A process according to claim 27 or 28, wherein R₂ is

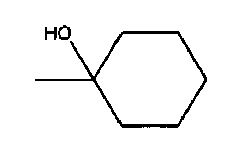

30. A process according to any one of the preceding claims, wherein the one or more cross-linking agent (3) is present in the mixture in step (i) in an amount of from about 0.01 % to about 0,5% by weight of the total polymerisation reaction mixture.

31. A process according to claim 30, wherein the cross-linking agent (3) is present in the mixture in step (i) in an amount of from about 0.05% to about 0.4% by weight of the total polymerisation reaction mixture.

32. A process according to claim 30, wherein the cross-linking agent (3) is present in the mixture in step (i) in an amount of from about 0.08% to about 0.3% by weight of the total polymerisation reaction mixture.

33. A process according to any one of the preceding claims, wherein the cross-linking agent comprises tripropylene glycol diacrylate, ethylene glycol dimethacrylate, triacrylate, polyethylene glycol diacrylate or methylene bis acrylamide.

34. A hydrogel composition prepared by a process according to any one of claims 1 to 33.

35. A product for containing or covering the skin, comprising a hydrogel composition according to claim 34.

36. A product according to claim 35, dimensioned and configured as an article selected from patches, tapes, bandages, devices and dressings of general utility or for specific biomedical, skin care, personal care, body care, palliative or veterinary uses.

37. A product according to claim 36, being an article selected from: skin electrodes; an article to assist wound and burn healing, wound and burn management, skin cooling, skin moisturising, skin warming, aroma release or delivery, decongestant release or delivery, pharmaceutical and drug release or delivery, perfume release or delivery, fragrance release or delivery, or scent release or delivery; an adhesive skin contacting device, or an adhesive ostomy or related incontinence device.

## Patentansprüche

1. Verfahren zur Herstellung einer vernetzten, schichtförmigen Hydrogelzusammensetzung mit hohem Wassergehalt, das folgende Schritte aufweist:
(i) Herstellen einer Mischung, welche Folgendes enthält:
(1) ein oder mehrere ungesättigte, radikalisch photopolymerisierbare Monomere, die mittels Polymerisation zu einem hydrophilen Polymer reagieren können;
(2) einen oder mehrere radikalische Photoinitiatoren;
(3) ein oder mehrere Vernetzungsmittel, die eine multifunktionale, ungesättigte, radikalisch photopolymerisierbare Verbindung aufweisen; sowie
(4) Wasser;
und
(ii) Bestrahlen der Mischung mit Licht von ausreichender Stärke und mit geeigneter Wellenlänge, um die Mischung zu polymerisieren und zu vernetzen und damit die plattenförmige Zusammensetzung zu bilden;
wobei alle der Verbindungen (1) bis (4), die in der Mischung in Schritt (i) vorhanden sind, auch in der Zusammensetzung vorhanden sind, die sich aus Schritt (ii) ergibt, der Photoinitiator (2) in der Mischung in Schritt (i) in einer Menge zwischen etwa 0,002 Gew.% und etwa 0,05 Gew.% der gesamten Mischung vorhanden ist, das Vernetzungsmittel (3) in der Mischung in Schritt (i) in einer Menge von weniger als etwa 0,5 Gew.% der gesamten Mischung vorhanden ist, und die entstehende Hydrogelzusammensetzung mehr als etwa 40 Gew.% Wasser enthält.

2. Verfahren nach Anspruch 1, bei dem die entstehende Hydrogelzusammensetzung mehr als etwa 50 Gew.% Wasser enthält.

3. Verfahren nach Anspruch 2, bei dem die entstehende Hydrogelzusammensetzung zwischen etwa 60 Gew.% und etwa 95 Gew.% Wasser enthält.

4. Verfahren nach einem der vorhergehenden Ansprüche, bei dem die in Schritt (i) hergestellte Mischung außerdem einen oder mehrere Elektrolyte aufweist.

5. Verfahren nach einem der vorhergehenden Ansprüche, bei dem die in Schritt (i) hergestellte Mischung außerdem einen oder mehrere organische Weichmacher aufweist.

6. Verfahren nach Anspruch 5, bei dem der organische Weichmacher einen der folgenden Stoffe allein oder in Kombination aufweist: mindestens einen mehrwertigen Alkohol, mindestens einen daraus gewonnenen Ester, mindestens einen polymeren Alkohol und/oder mindestens ein mono- oder polyalkyliertes Derivat eines polymeren Alkohols.

7. Verfahren nach Anspruch 5 oder 6, bei dem der organische Weichmacher Glycerol oder einen aus Borsäure und Glycerol gewonnenen Ester aufweist.

8. Verfahren nach einem der vorhergehenden Ansprüche, bei dem die in Schritt (i) hergestellte Mischung außerdem einen oder mehrere grenzflächenaktive Stoffe enthält.

9. Verfahren nach Anspruch 1, bei dem die in Schritt (i) hergestellte Mischung im Wesentlichen aus den Bestandteilen (1) bis (4) und wahlweise einem oder mehreren Elektrolyten und/oder einem oder mehreren organischen Weichmachern und/oder einem oder mehreren grenzflächenaktiven Stoffen besteht.

10. Verfahren nach einem der vorhergehenden Ansprüche, durchgeführt in kontinuierlicher Weise zur Produktion einer ununterbrochenen Hydrogelschicht.

11. Verfahren nach Anspruch 10, bei dem die Hydrogelschicht eine Dicke im Bereich von etwa 0,2mm bis etwa 2mm hat.

12. Verfahren nach einem der vorhergehenden Ansprüche, bei dem das eine oder die mehreren Monomere 2-Acrylamido-2-methylpropansulfonsäure, Acrylsäure, Acrylsäure-(3-sulfopropyl)ester, ein substituiertes Derivat derselben oder ein Salz derselben aufweisen.

13. Verfahren nach einem der vorhergehenden Ansprüche, bei dem das eine oder die mehreren Monomere mindestens einen der folgenden Stoffe aufweisen: Acrylamid oder ein mono- oder di-N-Alkylacrylamid oder ein Analogon davon, das eine Alkyl- oder substituierte Alkylgruppe aufweist, welche über eine Amido- oder Alkylamidofunktion mit einer Kohlenstoff-Kohlenstoff-Doppelbindung verbunden ist.

14. Verfahren nach Anspruch 13, bei dem das Analogon Diacetonacrylamid, ein Vinyllactam, ein N-alkyliertes Acrylamid, ein N,N-dialkyliertes Acrylamid, N-Vinylpyrrolidon oder Acryloylmorpholin ist.

15. Verfahren nach Anspruch 14, bei dem das Monomer Acryloylmorpholin ist.

16. Verfahren nach einem der vorhergehenden Ansprüche, bei dem die Polymerisation eine Homopolymerisation ist.

17. Verfahren nach einem der Ansprüche 1-15, bei dem die Polymerisation eine Copolymerisation ist.

18. Verfahren nach einem der vorhergehenden Ansprüche, bei dem der Monomerbestandteil (1) ein ionisches Monomer in einer Menge von 5 Gew.% bis 40 Gew.% der Mischung für die Polymerisationsreaktion aufweist.

19. Verfahren nach einem der vorhergehenden Ansprüche, bei dem der Monomerbestandteil (1) ein nichtionisches Monomer aufweist, das in einer Menge von bis zu 30 Gew.% der Mischung für die Polymerisationsreaktion vorhanden ist.

20. Verfahren nach einem der vorhergehenden Ansprüche, bei dem der Monomerbestandteil (1) in der Mischung für die Polymerisationsreaktion im Bereich von 5 Gew.% bis 40 Gew.% der gesamten Reaktionsmischung vorhanden ist.

21. Verfahren nach einem der vorhergehenden Ansprüche, bei dem nach dem Schritt (ii) im Wesentlichen kein Material der Zusammensetzung beigemengt oder entzogen wird.

22. Verfahren nach einem der vorhergehenden Ansprüche, bei dem nach der Herstellung dem Hydrogel kein Wasser entzogen wird und während der Herstellung weniger als 10% Wasser entzogen werden.

23. Verfahren nach einem der vorhergehenden Ansprüche, bei dem der Photoinitiator (2) in der Mischung in Schritt (i) in einer Menge von etwa 0,003 Gew.% bis etwa 0,05 Gew.% der gesamten Mischung für die Polymerisationsreaktion vorhanden ist.

24. Verfahren nach Anspruch 23, bei dem der Photoinitiator (2) in der Mischung in Schritt (i) in einer Menge von etwa 0,003 Gew.% bis etwa 0,04 Gew.% der gesamten Mischung für die Polymerisationsreaktion vorhanden ist.

25. Verfahren nach Anspruch 24, bei dem der Photoinitiator (2) in der Mischung in Schritt (i) in einer Menge von 0,003 Gew.% bis 0,02 Gew.% der gesamten Reaktionsmischung vorhanden ist.

26. Verfahren nach Anspruch 23, bei dem der Photoinitiator (2) in der Mischung in Schritt (i) in einer Menge von etwa 0,009 Gew.% bis etwa 0,02 Gew.% der gesamten Mischung für die Polymerisationsreaktion vorhanden ist.

27. Verfahren nach einem der vorhergehenden Ansprüche, bei dem der Photoinitiator eine Verbindung mit der folgenden allgemeinen Formel aufweist: 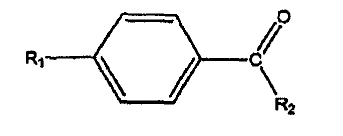 wobei R₁ ausgewählt wird aus Wasserstoff, H₃C-S-, 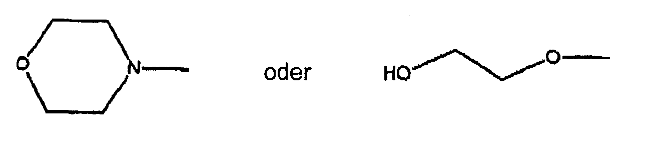 R₂ ausgewählt wird aus

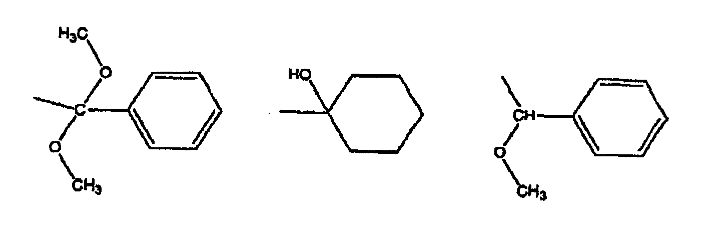

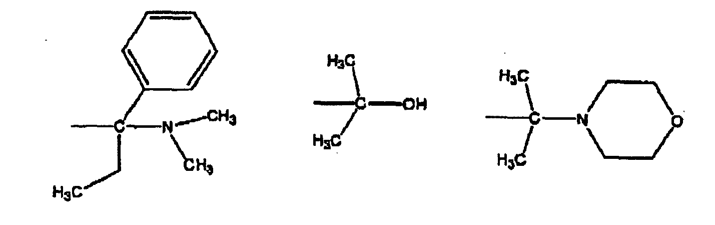

28. Verfahren nach Anspruch 27, bei dem R₁ Wasserstoff ist.

29. Verfahren nach Anspruch 27 oder 28, bei dem R₂ 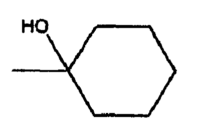 ist.

30. Verfahren nach einem der vorhergehenden Ansprüche, bei dem das eine oder die mehreren Vernetzungsmittel (3) in der Mischung in Schritt (i) in einer Menge von etwa 0,01 Gew.% bis etwa 0,5 Gew.% der gesamten Mischung für die Polymerisationsreaktion vorhanden sind.

31. Verfahren nach Anspruch 30, bei dem das Vernetzungsmittel (3) in der Mischung in Schritt (i) in einer Menge von etwa 0,05 Gew.% bis etwa 0,4 Gew.% der gesamten Mischung für die Polymerisationsreaktion vorhanden ist.

32. Verfahren nach Anspruch 30, bei dem das Vernetzungsmittel (3) in der Mischung in Schritt (i) in einer Menge von etwa 0,08 Gew.% bis etwa 0,3 Gew.% der gesamten Mischung für die Polymerisationsreaktion vorhanden ist.

33. Verfahren nach einem der vorhergehenden Ansprüche, bei dem das Vernetzungsmittel Tripropylenglykoldiacrylat, Ethylenglykoldimethacrylat, Triacrylat, Polyethylenglykoldiacrylat oder Methylen-bis-acrylamid aufweist.

34. Hydrogelzusammensetzung, die durch ein Verfahren nach einem der Ansprüche 1 bis 33 hergestellt wird.

35. Produkt zum Umschließen oder Abdecken der Haut, das eine Hydrogelzusammensetzung nach Anspruch 34 enthält.

36. Produkt nach Anspruch 35, das als ein Gegenstand dimensioniert und ausgebildet ist, der ausgewählt wird aus Pflastern, Streifen, Bandagen, Vorrichtungen und Verbänden für den allgemeinen Gebrauch oder für bestimmte biomedizinische, Hautpflege-, Hygiene-, Körperpflege-, palliative oder tierärztliche Anwendungen.

37. Produkt nach Anspruch 36, welches ein Gegenstand ist, der ausgewählt wird aus: Hautelektroden; einem Gegenstand, der beiträgt zur Heilung von Wunden und Verbrennungen, zur Behandlung von Wunden und Verbrennungen, zum Kühlen der Haut, zum Befeuchten der Haut, zum Erwärmen der Haut, zur Freisetzung oder Abgabe von Aromen, zur Freisetzung oder Abgabe von abschwellenden Mitteln, zur Freisetzung oder Abgabe von Arzneimitteln und Medikamenten, zur Freisetzung oder Abgabe von Parfum, zur Freisetzung oder Abgabe von Duftstoffen, oder zur Freisetzung oder Abgabe von Geruchsstoffen; einer Klebevorrichtung für den Kontakt mit der Haut, oder einer Klebevorrichtung für Ostomie- oder verwandte Inkontinenzanwendungen.

## Revendications

1. Procédé de production d'une composition d'hydrogel réticulé en feuille ayant une grande teneur en eau, comprenant :
(i) la préparation d'un mélange comprenant :
(1) un ou plusieurs monomères insaturés, photo-polymérisables par radicaux libres et aptes à se polymériser en un polymère hydrophile ;
(2) un ou plusieurs photoamorceurs à radicaux libres ;
(3) un ou plusieurs agents de réticulation comprenant un composé insaturé, multi-fonctionnel et photopolymérisable par radicaux libres ;
et
(4) de l'eau ; et
(ii) l'exposition du mélange à de la lumière d'une intensité suffisante et d'une longueur d'onde appropriée pour polymériser et réticuler le mélange pour former la composition en feuille ;
dans lequel tous les composés (1) à (4) présents dans le mélange au stade (i) sont présents aussi dans la composition obtenue dans le stade (ii), le photoamorceur (2), est présent dans le mélange au stade (i) en une quantité représentant entre environ 0,002 % et environ 0,05 % du poids du mélange total, l'agent (3) de réticulation est présent dans le mélange au stade (i) en une quantité représentant moins d'environ 0,5 % du poids du mélange total et la composition d'hydrogel obtenue comprend plus d'environ 40 % en poids d'eau.

2. Procédé suivant la revendication 1, dans lequel la composition d'hydrogel obtenue comprend plus d'environ 50 % en poids d'eau.

3. Procédé suivant la revendication 2, dans lequel la composition d'hydrogel obtenue comprend entre environ 60 % à environ 95 % en poids d'eau.

4. Procédé suivant l'une quelconque des revendications précédentes, dans lequel le mélange préparé au stade (i) comprend, en outre, un ou plusieurs électrolytes.

5. Procédé suivant l'une quelconque des revendications précédentes, dans lequel le mélange préparé au stade (i) comprend, en outre, un ou plusieurs plastifiants organiques.

6. Procédé suivant la revendication 5, dans lequel le plastifiant organique comprend l'un quelconque des suivants seuls ou en combinaison : au moins un polyalcool, au moins un ester en dérivant, au moins un alcool polymère et/ou au moins un dérivé mono-alcoylé ou poly-alcoylé d'un alcool polymère.

7. Procédé suivant la revendication 5 ou 6, dans lequel le plastifiant organique comprend du glycérol ou un ester dérivé de l'acide borique et du glycérol.

8. Procédé suivant l'une quelconque des revendications précédentes, dans lequel le mélange préparé au stade (i) comprend, en outre, un ou plusieurs agents tensioactifs.

9. Procédé suivant la revendication 1, dans lequel le mélange préparé au stade (i) consiste essentiellement en des constituants (1) à (4) et, éventuellement, en un ou en plusieurs électrolyses et/ou en un ou en plusieurs plastifiants organiques et/ou en un ou en plusieurs agents tensioactifs.

10. Procédé suivant l'une quelconque des revendications précédentes, lorsqu'il est effectué d'une manière continue pour la production d'une feuille continue d'hydrogel.

11. Procédé suivant la revendication 10, dans lequel la feuille d'hydrogel a une épaisseur comprise entre environ 0,2 mm et environ 2 mm.

12. Procédé suivant l'une quelconque des revendications précédentes, dans lequel le monomère, ou les plusieurs monomères, comprend de l'acide 2-acrylamido-2-méthyl-propane sulfonique, de l'acide acrylique, de l'ester (3-sulfopropylique) de l'acide acrylique, l'un de leurs dérivés substitués ou l'un de leurs sels.

13. Procédé suivant l'une quelconque des revendications précédentes, dans lequel le monomère, ou les plusieurs monomères, comprend au moins l'un d'un acrylamide ou d'un mono-alcoylacrylamide ou d'un di-N-alcoylacrylamide ou l'un de leurs analogues contenant un groupe alcoyle ou alcoyle substitué relié à une double liaison carbone-carbone par l'intermédiaire d'une fonction amido ou alcoylamido.

14. Procédé suivant la revendication 13, dans lequel l'analogue est un diacétone acrylamide, un vinyl lactame, un acrylamide N-alcoylé, un acrylamide N-dialcoylé, une N-vinyl pyrrolidone ou de l'acryloyl morpholine.

15. Procédé suivant la revendication 14, dans lequel le monomère est de l'acryloyl morpholine.

16. Procédé suivant l'une quelconque des revendications précédentes, dans lequel la polymérisation est une homopolymérisation.

17. Procédé suivant l'une quelconque des revendications précédentes, dans lequel la polymérisation est une copopolymérisation.

18. Procédé suivant l'une quelconque des revendications précédentes, dans lequel le constituant (1) monomère comprend un monomère ionique en une quantité représentant de 5 % à 40 % du poids du mélange de réaction de polymérisation.

19. Procédé suivant l'une quelconque des revendications précédentes, dans lequel le constituant (1) monomère comprend un monomère non ionique présent en une quantité représentant jusqu'à 30 % du poids du mélange de réaction de polymérisation.

20. Procédé suivant l'une quelconque des revendications précédentes, dans lequel le constituant (1) monomère est présent dans le mélange de réaction de polymérisation dans la plage de 5 % à 40 % du poids du mélange de réaction total.

21. Procédé suivant l'une quelconque des revendications précédentes, dans lequel de la matière n'est pas sensiblement ajoutée ou enlevée de la composition après le stade (ii).

22. Procédé suivant l'une quelconque des revendications précédentes, dans lequel de l'eau n'est pas éliminée de l'hydrogel après la production et moins de 10 % de l'eau est éliminée pendant la production.

23. Procédé suivant l'une quelconque des revendications précédentes, dans lequel le photoamorceur (2) est présent dans le mélange au stade (i) en une quantité représentant environ 0,003 % à environ 0,05 % du poids du mélange total de réaction de polymérisation.

24. Procédé suivant la revendication 23, dans lequel le photoamorceur (2) est présent dans le mélange au stade (i) en une quantité représentant environ 0,003 % à environ 0,04 % du poids du mélange total de réaction de polymérisation.

25. Procédé suivant la revendication 24, dans lequel le photoamorceur (2) est présent dans le mélange au stade (i) en une quantité représentant environ 0,003 % à environ 0,02 % du poids du mélange total de réaction de polymérisation.

26. Procédé suivant la revendication 23, dans lequel le photoamorceur (2) est présent dans le mélange au stade (i) en une quantité représentant environ 0,009 % à environ 0,02 % du poids du mélange total de réaction de polymérisation.

27. Procédé suivant l'une quelconque des revendications précédentes, dans lequel le photoamorceur comprend un composé de formule générale suivante : 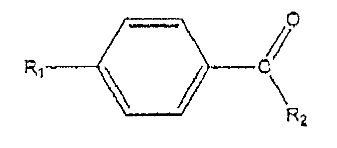 dans laquelle R₁ est choisi parmi hydrogène H₃C-S-, 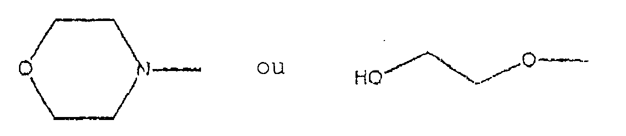 dans laquelle R₂ est choisi parmi

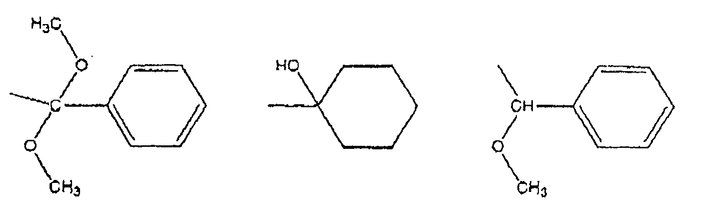

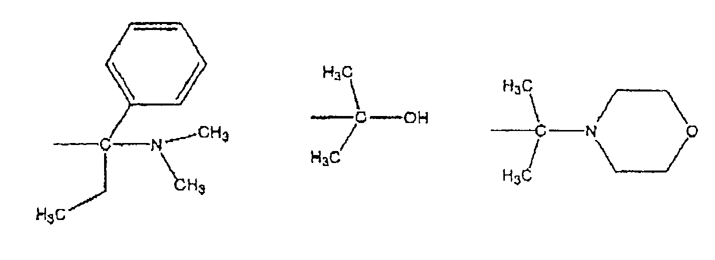

28. Procédé suivant la revendication 27, dans lequel R₁ est hydrogène.

29. Procédé suivant la revendication 27 ou 28, dans lequel R₂ est

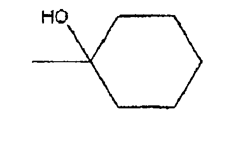

30. Procédé suivant l'une quelconque des revendications précédentes, dans lequel l'agent (3) de réticulation, ou les plusieurs agents (3) de réticulation, est présent dans le mélange au stade (i) en une quantité représentant environ 0,01 % à environ 0,5 % du poids du mélange total de réaction de polymérisation.

31. Procédé suivant la revendication 30, dans lequel l'agent (3) de réticulation ou les plusieurs agents (3) de réticulation est présent dans le mélange au stade (i) en une quantité représentant environ 0,05 % à environ 0,4 % du poids du mélange total de réaction de polymérisation.

32. Procédé suivant la revendication 30, dans lequel l'agent (3) de réticulation ou les plusieurs agents (3) de réticulation est présent dans le mélange au stade (i) en une quantité représentant environ 0,08 % à environ 0,3 % du poids du mélange total de réaction de polymérisation.

33. Procédé suivant l'une quelconque des revendications précédentes, dans lequel l'agent de réticulation comprend du diacrylate de tripropylène glycol, du diméthacrylate d'éthylène glycol, du triacrylate, du diacrylate de polyéthylène glycol ou du méthylène bis acrylamide.

34. Composition d'hydrogel préparé par un procédé suivant l'une quelconque des revendications 1 à 33.

35. Produit pour recouvrir la peau, comprenant une composition d'hydrogel suivant la revendication 34.

36. Produit suivant la revendication 35, dimensionné et configuré sous la forme d'un article choisi parmi des timbres, des rubans, des bandages, des dispositifs et des compresses d'utilité générale ou à des usages précis biomédicaux, de soin de la peau, de soin personnel, de soin du corps, palliatifs ou vétérinaires.

37. Produit suivant la revendication 36, qui est un article choisi parmi : des électrodes pour la peau ; un article pour soigner les blessures et guérir des brûlures, pour la gestion des blessures et des brûlures, pour le refroidissement de la peau, pour l'humidification de la peau, pour le réchauffement de la peau, pour la libération ou la distribution d'arôme, pour la libération ou la distribution d'agent de décongestion, pour la libération ou la distribution de produit pharmaceutique et de médicament, pour la libération ou la distribution de parfum, pour la libération ou la distribution de fragrance, ou pour la libération ou la distribution de senteur ; un dispositif adhésif de contact avec la peau, ou un dispositif adhésif d'ostomie ou d'incontinence apparenté.
